(19)

Europäisches
Patentamt
European
Patent Office
Office européen
des brevets

(11) **EP 2 503 318 A2**

(12) **EUROPEAN PATENT APPLICATION**

(43) Date of publication:
**26.09.2012 Bulletin 2012/39**

(51) Int Cl.:
***G01N 9/00*** *(2006.01)*

(21) Application number: **12156463.7**

(22) Date of filing: **22.02.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **24.03.2011 JP 2011066579**

(71) Applicant: **Azbil Corporation
Chiyoda-ku
Tokyo (JP)**

(72) Inventor: **Ooishi, Yasuharu
Tokyo, 100-6419 (JP)**

(74) Representative: **Tischner, Oliver
Lavoix Munich
Bayerstrasse 85a
80335 Munich (DE)**

(54) **Density measuring system and density measuring method**

(57)     A density measuring system, including a container into which a gas is injected, provided with a heating element to which a variety of different voltages is applied; an equation storage device storing a density calculating equation that has, as independent variables, electrical signals obtained from the heating element when each of the plurality of different voltages is applied and has the density as a dependent variable; and a density calculating portion calculating a measured value for the density of the gas that is injected into the container, through substituting measured values for the electrical signals from the heating element into the independent variables of the density calculating equation.

**FIG. 6**

EP 2 503 318 A2

**Description**

Cross Reference to Related Applications

The present application claims priority to Japanese Patent Application No. 2011-066579, filed March 24, 2011, which is incorporated herein by reference.

Field of Technology

The present invention relates to a density measuring system and density measuring method relating to a gas testing technology.

Background

Oscillating-type gas density meters are known as means for measuring densities of gases. An oscillating-type gas density meter makes use of variance in the resonant frequency of a cylindrical oscillator, where this variance is dependent on the density of the ambient gas, to measure the density of the gas. Consequently, an oscillating-type gas density meter has a drawback in that it cannot measure the density of the gas accurately in the presence of an external vibration. Because of this, there have been proposals for supporting the cylindrical oscillator on an elastic body (referencing, for example, Japanese Unexamined Patent Application Publication H10-281967).
However, even when the cylindrical oscillator supported on an elastic body, still the oscillation-type gas density meter is unable to measure the gas density accurately when in the presence of a strong vibration from the outside. Given this, one object of the present invention is the provision of a density measuring system and density measuring method whereby the density of a gas can be measured easily and accurately.

Summary

In the light of above, a density measuring system according to claim 1 and a method for measuring a desity of a gas according to claim 8 are provided.
An example of the present invention provides a density measuring system having (a) a container, into which a gas is injected, provided with a heating element to which a variety of different voltages is applied; (b) a memory device for storing a density calculating equation that has, as independent variables, electrical signals obtained from the heating element when each of the plurality of different voltages is applied and has the density as a dependent variable; and (c) a density calculating portion for calculating a measured value for the density of the gas that is injected into the container, through substituting measured values for the electrical signals from the heating element into the independent variables of the density calculating equation.
Moreover, another example of the present invention provides a density measuring method that includes (a) an injection of a gas into a container that is provided with a heating element to which a variety of different voltages is applied; (b) a provision of a density calculating equation that has, as independent variables, electrical signals obtained from the heating element when each of the plurality of different voltages is applied and has the density as a dependent variable; and (c) a calculation of a measured value for the density of the gas that is injected into the container, through substituting measured values for the electrical signals from the heating element into the independent variables of the density calculating equation.
A further example of the present invention provides a density measuring system having (a) a measuring portion for measuring a measured value of a gas radiation coefficient or a thermal conductivity; (b) a storage device for storing a correlation between a radiation coefficient or a thermal conductivity and a density; and (c) a density calculating portion for calculating a measured value for a density of a gas based on the measured value for the radiation coefficient or thermal conductivity of the gas and the correlation. The variates in the correlation may include density. In this case, the density calculating portion may calculate the measured value of the density of the gas based on a measured value of the pressure of the gas, a measured value of a radiation coefficient or a thermal conductivity of the gas, and a correlation.
Yet another example of the present invention provides a density measuring method that includes (a) a measurement of a measured value of a gas radiation coefficient or a thermal conductivity; (b) a preparation of a correlation between a radiation coefficient or a thermal conductivity and a density; and (c) a calculation of a measured value for a density of a gas based on the measured value for the radiation coefficient or thermal conductivity and the correlation. The variates in the correlation may include density. In this case, the calculation of the measured value of the density of the gas may be based on a measured value of the pressure of the gas, a measured value of a radiation coefficient or a thermal conductivity of the gas, and a correlation.
According to an embodiment, a density measuring system is provided, including a container into which a gas is injected, provided with a heating element to which a variety of different voltages is applied; an equation storage device storing a density calculating equation that has, as independent variables, electrical signals obtained from the heating element when each of the plurality of different voltages is applied and has the density as a dependent variable; and a density calculating portion calculating a measured value for the density of the gas that is injected into the container, through substituting measured values for the electrical signals from the heating element into the independent variables of the density calculating equation.

The present invention enables the provision of a density measuring system and a density measuring method able to measure the density of a gas easily and accurately. Further advantages, features, aspects and details are evident from the dependent claims, the description and the drawings, which relate to embodiments of the invention and are described in the following:

Brief Description of the Drawings

FIG. 1 is a perspective view of a microchip as set forth in an example.
FIG. 2 is a cross-sectional diagram, viewed from the direction of the section II-II, of the microchip as set forth in the example.
FIG. 3 is a circuit diagram relating to a heating element according to an example of the present invention.
FIG. 4 is a circuit diagram relating to a temperature measuring element according to another example of the present invention.
FIG. 5 is a graph illustrating the relationship between the temperature of the heating element and the radiation coefficient of the gas.
FIG. 6 is a schematic diagram of a density measuring system as set forth in an example of the present invention.
FIG. 7 is a schematic diagram of a density measuring system as set forth in the example.
FIG. 8 is a flowchart illustrating a method for generating a density calculating equation as set forth in a further example.
FIG. 9 is a flowchart illustrating a density measuring method as set forth in another example.
FIG. 10 is a graph illustrating the relationship between the gas thermal conductivity and heat loss coefficient in yet another example.
FIG. 11 is a graph showing the errors from the true value of densities calculated for a sample mixed gas relating to an example of the present invention.
FIG. 12 is a graph showing the errors from the true values of calorific values calculated for a sample mixed gas relating to an example.

Detailed Description

Examples of the present invention are described below. In the descriptions of the drawings below, identical or similar components are indicated by identical or similar codes. Note that the diagrams are schematic. Consequently, specific measurements should be evaluated in light of the descriptions below. Furthermore, even within these drawings there may, of course, be portions having differing dimensional relationships and proportions.

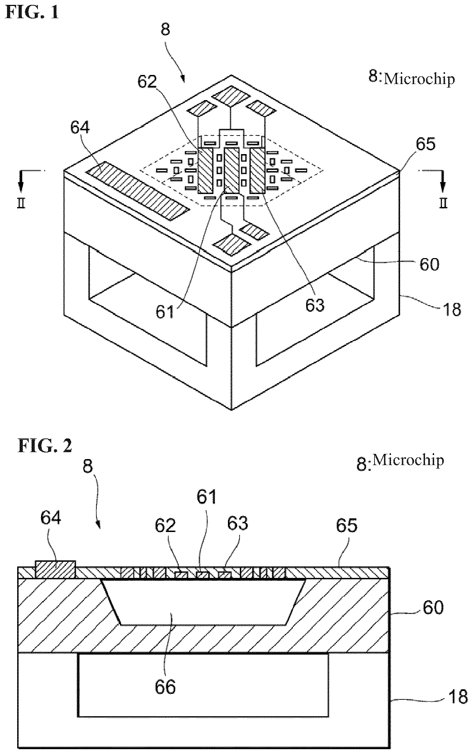

First a microchip 8 that is used in a density measuring system as set forth in an embodiment is described in reference to FIG. 1, which is a perspective diagram, and FIG. 2, which is a cross-sectional diagram that is viewed from the direction of the section II-II. The microchip 8 comprises a substrate 60, which is provided with a cavity 66, and a dielectric layer 65, which is disposed so as to cover the cavity 66 on the substrate 60. The thickness of the substrate 60 is, for example, 0.5 mm. The length and width dimensions of the substrate 60 are, for example, 1.5 mm each. The portion of the dielectric layer 65 that covers the cavity 66 forms a thermally insulating diaphragm. The microchip 8 further comprises a heating element 61 that is provided on a portion of the diaphragm of the dielectric layer 65, a first temperature measuring element 62 and a second temperature measuring element 63 provided in a portion of the diaphragm of the dielectric layer 65 so that the heating element 61 is interposed therebetween, and a temperature maintaining element 64 that is provided on the substrate 60.

The heating element 61 is disposed in the center of the portion of the diaphragm of the dielectric layer 65 that covers the cavity 66. The heating element 61 is, for example, a resistor, and produces heat through the supply of electric power thereto, to heat the ambient gas that contacts the heating element 61. The first temperature measuring element 62 and the second temperature measuring element 63 are electrical elements that are, for example, passive elements such as resistors, and output electric signals that are dependent on the gas temperatures of the surrounding gases. An example of use of the output signal of the first temperature measuring element 62 is explained below, but there is no limitation thereto, but rather, for example, an average value of the output signal from the first temperature measuring element 62 and the output signal of the second temperature measuring element 63 may be used as the output signal of the temperature measuring elements.

The temperature maintaining element 64 is, for example, a resistor, to which electricity is applied to produce heat, to maintain the substrate 60 at a constant temperature. Silicon (Si), or the like, may be used as the material for the substrate 60. Silicon dioxide ($SiO_2$), or the like, may be used as the material for the dielectric layer 65. The cavity 66 may be formed through anisotropic etching, or the like. Furthermore, platinum (Pt) or the like may be used as the material for the first temperature measuring element 62, the second temperature measuring element 63, and the temperature maintaining element 64, and they may be formed through a lithographic method, or the like. Moreover, the heating element 61, the first temperature measuring element 62, and the second temperature measuring element 63 may be formed from the same member.

The microchip 8 is secured, for example, to a container, such as a chamber, chamber, or the like, that is filled with the ambient gas, through, for example, a thermally insulating member that is disposed on the bottom face of the microchip 8. Securing the microchip 8 through a thermally insulating member 18 within a chamber, or the like, makes the temperature of the microchip 8 less susceptible to temperature variations of the inner wall of the chamber, or the like. The thermal conductivity of the insulating member 18, made from glass, or the like, is, for example, no more than 1.0 W/(m ·K).

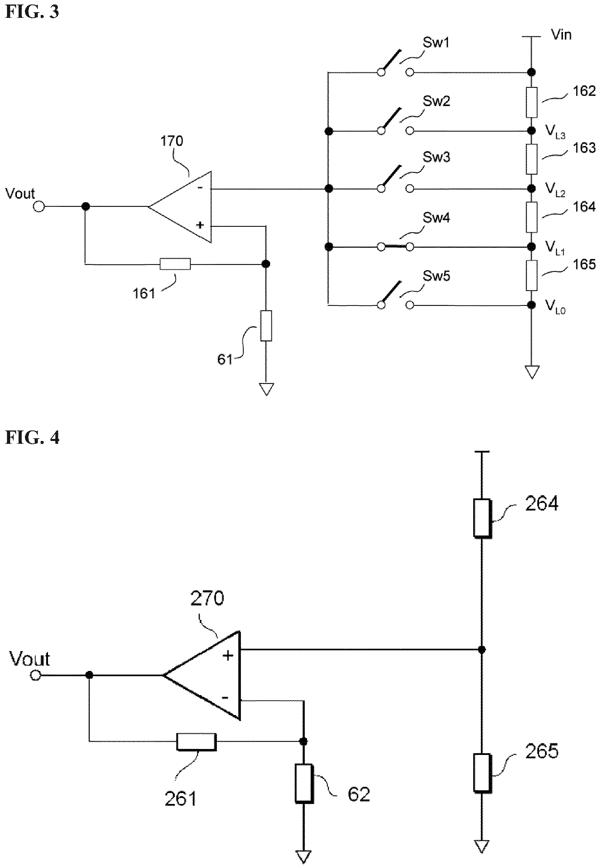

As illustrated in FIG. 3, one end of the heating element 61 is connected electrically to a + input terminal of an operational amplifier 170, for example, with the other end grounded. A resistive element 161 is connected, in parallel, to the + input terminal and the output terminal of the operational amplifier 170. The - input terminal of the operational amplifier 170 is connected electrically to a power supply, between a resistive element 162 and a resistive element 163, which are connected in series, between the resistive element 163 and a resistive element 164, which are connected in series, between the resistive element 164 and a resistive element 165, which are connected in series, or between the resistive element 165 and a ground terminal. The appropriate selection of the resistance values for each of the resistive elements 162 through 165 will produce a voltage $V_{L3}$ of, for example, 2.8 V between the resistive element 163 and the resistive element 162 when a voltage Vin of, for example, 3.2 V is applied to one end of the resistive element 162. Moreover, a voltage $V_{L2}$ of, for example, 2.2 V is produced between the resistive element 164 and resistive element 163, and a voltage $V_{L1}$ of, for example, 1.5 V is produced between the resistive element 165 and resistive element 164.

A switch SW1 is provided between the power supply and the - input terminal of the operational amplifier 170, a switch SW2 is provided between the connection between the resistive element 162 and the resistive element 163 and the - input terminal of the operational amplifier 170, and a switch SW3 is provided between the connection between the resistive element 163 and the resistive element 164 and the - input terminal of the operational amplifier 170. Furthermore, a switch SW4 is provided between the connection between the resistive element 164 and the resistive element 165 and the - input terminal of the operational amplifier 170, and a switch SW5 is provided between the connection between the resistive element 165 and ground terminal and the - input terminal of the operational amplifier 170.

When applying a 3.2 V voltage Vin to the - input terminal of the operational amplifier 170, only the switch SW1 is conductive, and the switches SW2, SW3, SW4, and SW5 are non-conductive. When applying a 2.8 V voltage $V_{L3}$ to the - input terminal of the operational amplifier 170, only the switch SW2 is conductive, and the switches SW1, SW3, SW4, and SW5 are non-conductive. When a applying 2.2 V voltage $V_{L2}$ to the - input terminal of the operational amplifier 170, only the switch SW3 is conductive, and the switches SW1, SW2, SW4, and SW5 are non-conductive. When applying a 1.5 V voltage $V_{L1}$ to the - input terminal of the operational amplifier 170, only the switch SW4 is conductive, and the switches SW1, SW2, SW3, and SW5 are non-conductive. When a applying 0 V voltage $V_{L0}$ to the - input terminal of the operational amplifier 170, only the switch SW5 is conductive, and the switches SW1, SW2, SW3, and SW4 are non-conductive. Consequently, either 0 V or any of four different voltage levels may be applied to the - input terminal of the operational amplifier 170, depending on the open/closed statuses of the switches SW1, SW2, SW3, SW4, and SW5. Because of this, the applied voltages, which determine the heat producing temperature of the heating element 61, can be set through opening and closing the switches SW1, SW2, SW3, SW4, and SW5.

Here the temperature of the heating element 61 when the 1.5 V voltage VL1 is applied to the - input terminal of the operational amplifier 170 is defined as TH1. Additionally, the temperature of the heating element 61 when the 2.2 V voltage VL2 is applied to the - input terminal of the operational amplifier 170 is defined as TH2, and the temperature of the heating element 61 when the 2.8 V voltage VL3 is applied to the - input terminal of the operational amplifier 170 is defined as $T_{H3}$.

As illustrated in FIG. 4, one end of the first temperature measuring element 62 is connected electrically to a - input terminal of an operational amplifier 270, for example, with the other end grounded. A resistive element 261 is connected, in parallel, to the - input terminal and the output terminal of the operational amplifier 270. The + input terminal of the operational amplifier 270 is connected electrically to between a resistive element 264 and a resistive element 265 that are connected in series. This causes a weak voltage of about 0.3 V to be applied to the first temperature measuring element 62.

The resistance value of the heating element 61 illustrated in FIG. 1 and FIG. 2 varies depending on the temperature of the heating element 61. The relationship between the temperature $T_H$ of the heating element 61 and the resistance value $R_H$ of the heating element 61 is given through Equation (1), below:

$$R_H = R_{H\_STD} \times [1 + \alpha_H (T_H - T_{H\_STD}) + \beta_H (T_H - T_{H\_STD})^2] \ldots (1)$$

Here $T_{H\_STD}$ indicates a standard temperature for the heating element 61 of, for example, 20°C. $R_{H\_STD}$ indicates the resistance value of the heating element 61 measured in advance at the standards temperature of $T_{H\_STD}$. $\alpha_H$ indicates a first-order resistance temperature coefficient. $\beta_H$ indicates a second-order resistance temperature coefficient.

The resistance value $R_H$ of the heating element 61 is given by Equation (2), below, from the driving power $P_H$ of the heating element 61 and the current $I_H$ that flows through the heating element 61.

$$R_H = P_H / I_H{}^2 \ldots (2)$$

Conversely, the resistance value $R_H$ of the heating element 61 is given by Equation (3), below, from the voltage $V_H$ applied to the heating element 61 and the current $I_H$ that flows through the heating element 61.

$$R_H = V_H / I_H \ldots (3)$$

Here the temperature $T_H$ of the heating element 61 reaches a thermal equilibrium and stabilizes between the heating element 61 and the ambient gas. Note that this "thermal equilibrium" refers to a state wherein there is a balance between the heat production by the heating element 61 and the heat dissipation from the heating element 61 into the ambient gas. As shown in Equation (4), below, the driving power $P_H$ of the heating element 61 in the state of thermal equilibrium is divided by the difference $\Delta T_H$ between the temperature $T_H$ of the heating element 61 and the temperature $T_I$ of the ambient gas, to produce the radiation coefficient $M_I$ of the ambient gas. Note that the units for the radiation coefficient $M_I$ are, for example, W/°C.

$$M_I = P_H / (T_H - T_I)$$
$$= P_H / \Delta T_H \ldots (4)$$

From Equation (1), above, the temperature $T_H$ of the heating element 61 is obtained through Equation (5), below:

$$T_H = (1 / 2\beta_H) \times [-\alpha_H + [\alpha_H{}^2 - 4\beta_H (1 - R_H / R_{H\_STD})]^{1/2}] + T_{H\_STD} \ldots (5)$$

Consequently, the difference $\Delta T_H$ between the temperature $T_H$ of the heating element 61 and the temperature $T_I$ of the ambient gas is given by Equation (6), below:

$$\Delta T_H = (1 / 2\beta_H) \times [-\alpha_H + [\alpha_H{}^2 - 4\beta_H (1 - R_H / R_{H\_STD})]^{1/2}] + T_{H\_STD} - T_I \ldots (6)$$

The temperature $T_I$ of the ambient gas temperature $T_I$ is approximated by the temperature $T_I$ of the first temperature measuring element 62 when power is applied to the extent that it does not produce heat itself. The relationship between the temperature $T_I$ of the first temperature measuring element 62 and the resistance value $R_I$ of the first temperature measuring element 62 is given by Equation (7), below:

$$R_I = R_{I\_STD} \times [1 + \alpha_I (T_I - T_{I\_STD}) + \beta_I (T_I - T_{I\_STD})^2] \ldots (7)$$

Here $T_{I\_STD}$ indicates a standard temperature for the first temperature measuring element 62 of, for example, 20°C. $R_{I\_STD}$ indicates the resistance value of the first temperature measuring element 62, measured in advance at the standard temperature of $T_{I\_STD}$. $A_I$ indicates a first-order resistance temperature coefficient. $B_I$ indicates a second-order resistance temperature coefficient. Through Equation (7), above, the temperature $T_I$ of the first temperature measuring element 62 is given by Equation (8), below:

$$T_I = (1 / 2\beta_I) \text{ x } [-\alpha_I + [\alpha_I{}^2 - 4\beta_I(1 - R_I / R_{I\_STD})]^{1/2}] + T_{I\_STD} \dots (8)$$

Consequently, the radiation coefficient $M_I$ of the ambient gas is given by Equation (9), below.

$$M_I = P_H / \Delta T_H$$
$$= P_H[(1/2\beta_H)[-\alpha_H + [\alpha_H{}^2 - 4\beta_H (1 - R_H/R_{H\_STD})]^{1/2}] + T_{H\_STD} - (1/2\beta_I)[-\alpha_I + [\alpha_I{}^2 - 4\beta_I (1 - R_I/R_{I\_STD})]^{1/2}] - T_{I\_STD}]$$
$$= (R_H/I_H{}^2)[(1/2\beta_H)[-\alpha_H + [\alpha_H{}^2 - 4\beta_H (1 - R_H/R_{H\_STD})]^{1/2}] + T_{H\_STD} - (1/2\beta_I)[-\alpha_I + [\alpha_I{}^2 - 4\beta_I (1 - R_I/R_{I\_STD})]^{1/2}] - T_{I\_STD}] \dots (9)$$

The electric current $I_H$ that flows in the heating element 61 and the driving power $P_H$ or the voltage $V_H$ can be measured, and thus the resistance value $R_H$ of the heating element 61 can be calculated from Equation (2) or Equation (3), above. Similarly, it is also possible to calculate the resistance value $R_I$ of the first temperature measuring element 62. Consequently, the radiation coefficient $M_I$ of the ambient gas can be calculated from Equation (9), above, using the microchip 8.

Note that holding the temperature of the substrate 60 constant, using the temperature maintaining element 64, causes the temperature of the ambient gas in the vicinity of the microchip 8, prior to heating by the heating element 61, to approximate the constant temperature of the substrate 60. This suppresses the variation in the temperature of the ambient gas prior to heating by the heating element 61. Further heating, by the heating element 61, the ambient gas for which the temperature variation had been controlled makes it possible to calculate the radiation coefficient $M_I$ with greater accuracy.

Here the ambient gas is a mixed gas, where the mixed gas is assumed to comprise four gas components: gas A, gas B, gas C, and gas D. The total of the volume fraction $V_A$ of the gas A, the volume fraction $V_B$ of the gas B, the volume fraction $V_C$ of the gas C, and the volume fraction $V_D$ of the gas D, as obtained by Equation (10), below, is 1.

$$V_A + V_B + V_C + V_D = 1 \dots (10)$$

Moreover, when the per-unit-volume calorific value of gas A is defined as $K_A$, the per-unit-volume calorific value of gas B is defined as $K_B$, the per-unit-volume calorific value of gas C is defined as $K_C$, and the per-unit-volume calorific value of gas D is defined as $K_D$, then the per-unit-volume calorific value Q of mixed gas is obtained by summing the products of the volume fractions of the individual gas components and the per-unit-volume calorific values of the individual gas components. Consequently, the per-unit-volume calorific value Q of the mixed gas is given by Equation (11), below. Note that the units for the per-unit-volume calorific values are, for example, $MJ/m^3$.

$$Q = K_A \text{ x } V_A + K_B \text{ x } V_B + K_C \text{ x } V_C + K_D \text{ x } V_D \dots (11)$$

Moreover, when the radiation coefficient of gas A is defined as $M_A$, the radiation coefficient of gas B is defined as $M_B$, the radiation coefficient of gas C is defined as $M_C$, and the radiation coefficient of gas D is defined as $M_D$, then the radiation coefficient of the mixed gas $M_I$ is given by summing the products of the volume fractions of the individual gas components and the radiation coefficients of the individual gas components. Consequently, the radiation coefficient $M_I$ of the mixed gas is given by Equation (12), below.

$$M_I = M_A \times V_A + M_B \times V_B + M_C \times V_C + M_D \times V_D \dots (12)$$

Moreover, because the radiation coefficient of the gas is dependent on the temperature $T_H$ of the heating element 61, the radiation coefficient $M_I$ of the mixed gas is given by Equation (13) as a function of the temperature $T_H$ of the heating element 61:

$$M_I(T_H) = M_A(T_H) \times_A + M_B(T_H) \times V_B + M_C(T_H) \times V_C + M_D(T_H) \times V_D \dots (13)$$

Consequently, when the temperature of the heating element 61 is $T_{H1}$, then the radiation coefficient $M_{I1}(T_{H1})$ of the mixed gas is given by Equation (14), below. Moreover, when the temperature of the heating element 61 is $T_{H2}$, then the radiation coefficient $M_{I2}(T_{H2})$ of the mixed gas is given by Equation (15), below, and when the temperature of the heating element 61 is $T_{H3}$, then the radiation coefficient $M_{I3}(T_{H3})$ of the mixed gas is given by Equation (16), below.

$$M_{I1}(T_{H1}) = M_A(T_{H1}) \times V_A + M_B(T_{H1}) \times V_B + M_C(T_{H1}) \times V_C + M_D(T_{H1}) \times V_D \dots (14)$$

$$M_{I2}(T_{H2}) = M_A(T_{H2}) \times V_A + M_B(T_{H2}) \times V_B + M_C(T_{H2}) \times V_C + M_D(T_{H2}) \times V_D \dots (15)$$

$$M_{I3}(T_{H3}) = M_A(T_{H3}) \times V_A + M_B(T_{H3}) \times V_B + M_C(T_{H3}) \times V_C + M_D(T_{H3}) \times V_D \dots (16)$$

If here the radiation coefficients $M_A(T_H)$, $M_B(T_H)$, $M_C(T_H)$, and $M_D(T_H)$ of the individual gas components are non-linear in respect to the temperature $T_H$ of the heating element 61, then the Equations (14) through (16), above, will have linearly independent relationships. Moreover, even if the radiation coefficients $M_A(T_H)$, $M_B(T_H)$, $M_C(T_H)$, and $M_D(T_H)$ of the individual gas components are linear in respect to the temperature $T_H$ of the heating element 61, if the rates of change of the radiation coefficients $M_A(T_H)$, $M_B(T_H)$, $M_C(T_H)$, and $M_D(T_H)$ of the individual gas components are non-linear in respect to the temperature $T_H$ of the heating element 61 the Equations (14) through (16), above, will have linearly independent relationships. Moreover, if Equations (14) through (16) have a linearly independent relationship, then Equation (10) and Equations (14) through (16) will have a linearly independent relationship.

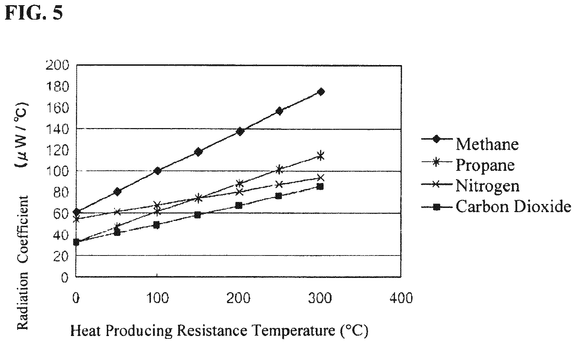

FIG. 5 is a graph showing the relationships of the radiation coefficients of methane ($CH_4$), propane ($C_3H_8$), nitrogen ($N_2$), and carbon dioxide ($CO_2$), which are included in natural gas, to the temperature of the heating element 61 which is a heat producing resistance. The radiation coefficients of each of these components (methane ($CH_4$), propane ($C_3H_8$), nitrogen ($N_2$), and carbon dioxide ($CO_2$)) are linear in respect to the temperature of the heating element 61. However, the respective rates of change of the radiation coefficients in respect to the temperature of the heating element 61 are different for methane ($CH_4$), propane ($C_3H_8$), nitrogen ($N_2$), and carbon dioxide ($CO_2$). Consequently, Equations (14) through (16), above, will be linearly independent if the gas components that comprise the mixed gas are methane ($CH_4$), propane ($C_3H_8$), nitrogen ($N_2$), and carbon dioxide ($CO_2$).

The values for the radiation coefficients $M_A(T_{H1})$, $M_B(T_{H1})$, $MC(T_{H1})$, $M_D(T_{H1})$, $M_A(T_{H2})$, $Ms(T_H2)$, $M_C(T_H2)$, $M_D(T_{H2})$, $M_A(T_{H3})$, $M_B(T_{H3})$, $M_C(T_H3)$, $M_D(T_{H3})$ for the individual gas components in Equation (14) through Equation (16) can be obtained in advance through measurements, or the like. Consequently, when the system of simultaneous equations of Equation (10) and Equation (14) through Equation (16) is solved, the volume fraction $V_A$ of the gas A" the volume fraction $V_B$ of the gas B, the volume fraction $V_C$ of the gas C, and the volume fraction $V_D$ of the gas D, respectively, are obtained as functions of the radiation coefficients $M_{I1}(T_{H1})$, $M_{I2}(T_{H2})$, and $M_{I3}(T_{H3})$ of the mixed gas. Note that in Equations (17) through (20), below, $f_n$, where n is a non-negative integer, is a code indicating a function:

$$V_A = f_1[M_{I1}(T_{H1}), M_{I2}(T_{H2}), M_{I3}(T_{H3})] \dots (17)$$

$$V_B = f_2[M_{I1}(T_{H1}), M_{I2}(T_{H2}), M_{I3}(T_{H3})] \dots (18)$$

$$V_C = f_3[M_{I1}(T_{H1}), M_{I2}(T_{H2}), M_{I3}(T_{H3})] \dots (19)$$

$$V_D = f_4[M_{I1}(T_{H1}), M_{I2}(T_{H2}), M_{I3}(T_{H3})] \dots (20)$$

Here Equation (21), below, is obtained through substituting Equation (17) through (20) into Equation (11), above.

$$Q = K_A \times V_A + K_B \times V_B + K_C \times V_C + K_D \times V_D$$
$$= K_A \times f_1[M_{I1}(T_{H1}), M_{I2}(T_{H2}), M_{I3}(T_{H3})]$$
$$+ K_B \times f_2[M_{I1}(T_{H1}), M_{I2}(T_{H2}), M_{I3}(T_{H3})]$$
$$+ K_C \times f_3[M_{I1}(T_{H1}), M_{I2}(T_{H2}), M_{I3}(T_{H3})]$$
$$+ K_D \times f_4[M_{I1}(T_{H1}), M_{I2}(T_{H2}), M_{I3}(T_{H3})] \dots (21)$$

As shown in Equation (21), above, the per-unit-volume calorific value Q is obtained as an equation which has, as variables, the radiation coefficients $M_{I1}(T_{H1})$, $M_{I2}(T_{H2})$, and $M_{I3}(T_{H3})$ of the mixed gas when the temperatures of the heating element 61 are $T_{H1}$, $T_{H2}$, and $T_{H3}$. Consequently, the calorific value Q of the mixed gas is given by Equation (22), below, where g is a code indicating a function.

$$Q = g[M_{I1}(T_{H1}), M_{I2}(T_{H2}), M_{I3}(T_{H3})] \dots (22)$$

Consequently, the inventors discovered that, for a mixed gas comprising a gas A, a gas D, a gas C, and a gas D, wherein the volume fraction $V_A$ of the gas A, the volume fraction $V_B$ of the gas B, the volume fraction $V_C$ of the gas C, and the volume fraction $V_D$ of the gas D, are unknown, it is possible to calculate easily the per-unit-volume calorific value of the mixed gas to be measured if Equation (22) is obtained in advance. Specifically, it is possible to calculate uniquely the calorific value Q of the mixed gas to be measured, through measuring the respective radiation coefficients $M_{I1}(T_{H1})$, $M_{I2}(T_{H2})$, and $M_{I3}(T_{H3})$ for the mixed gas to be measured, at the heat producing temperatures of $T_{H1}$, $T_{H2}$, and $T_{H3}$ of the heating element 61 and then substituting, into Equation (22).

Moreover, the thermal characteristics of a gas, such as the calorific value, the radiation coefficient, the thermal conductivity, and the like, are dependent on the pressure of the gas. Consequently, adding the independent variable of the pressure Ps of the mixed gas to be measured, as shown in Equation (23), below, to the formula for Q, obtained by Equation (22), above, increases the accuracy of the calculation of the calorific value Q.

$$Q = g[M_{I1}(T_{H1}), M_{I2}(T_{H2}), M_{I3}(T_{H3}), Ps] \dots (23)$$

Additionally, the radiation coefficient $M_I$ of the mixed gas, as indicated in Equation (9), above, depends on the resistance value $R_H$ of the heating element 61 and on the resistance value $R_I$ of the first temperature measuring element 62. Given this, the inventors discovered that the per-unit-volume calorific value Q of the thermal diffusion rate of a mixed gas can also be obtained from an equation having, as variables, the resistances $R_{H1}(T_{H1})$, $R_{H2}(T_{H2})$, and $R_{H3}(T_{H3})$ of the heating element 61 when the temperatures of the heating element 61 are $T_{H1}$, $T_{H2}$, and $T_{H3}$, the resistance value $R_I$ of the first temperature measuring element 62 that is in contact with the mixed gas, and the pressure Ps as shown in Equation (24), below.

$$Q = g[R_{H1}(T_{H1}), R_{H2}(T_{H2}), R_{H3}(T_{H3}), R_I, Ps] \dots (24)$$

Given this, the caloric content Q of a mixed gas to be measured can be calculated uniquely also by substituting, into Equation 24, the resistances $R_{H1}(T_{H1})$, $R_{H2}(T_{H2})$, and $R_{H3}(T_{H3})$ of the heating element 61 when the heat producing temperatures of the heating element 61, which is in contact with the mixed gas to be measured, are $T_{H1}$, $T_{H2}$, and $T_{H3}$, the resistance value $R_I$ of the first temperature measuring element 62 that is in contact with the mixed gas, and the pressure Ps of the mixed gas to be measured.

Moreover, the per-unit-volume calorific value Q of the thermal diffusion rate of a mixed gas can also be obtained from an equation having, as variables, the electric currents $I_{H1}(T_{H1})$, $I_{H2}(T_{H2})$, and $I_{H3}(T_{H3})$ in the heating element 61 when the temperatures of the heating element 61 are $T_{H1}$, $T_{H2}$, and $T_{H3}$, the electric current $I_I$ of the first temperature measuring element 62 that is in contact with the mixed gas, and the pressure Ps of the mixed gas, as shown in Equation (25), below.

$$Q = g[I_{H1} (T_{H1}), I_{H2} (T_{H2}), I_{H3} (T_{H3}), I_I, Ps ] \dots(25)$$

Conversely, the per-unit-volume calorific value Q of the thermal diffusion rate of a mixed gas can also be obtained from an equation having, as variables, the voltages $I_{H1}(T_{H1})$, $I_{H2}(T_{H2})$, and $I_{H3}(T_{H3})$ applied to heating element 61 when the temperatures of the heating element 61 are $T_{H1}$, $T_{H2}$, and $T_{H3}$, the voltage $V_I$ of the first temperature measuring element 62 that is in contact with the mixed gas, and the pressure Ps of the mixed gas, as shown in Equation (26), below.

$$Q = g[V_{H1} (T_{H1}), V_{H2} (T_{H2}), V_{H3} (T_{H3}), V_I, Ps ] \dots(26)$$

Conversely, the per-unit-volume calorific value of a mixed gas can also be obtained from an equation having, as variables, the output voltages $AD_{H1}(T_{H1})$, $AD_{H2}(T_{H2})$, and $AD_{H3}(T_{H3})$ of analog-digital converting circuits (hereinafter termed "A/D converting circuits") that are connected to the heating element 61 when the temperatures of the heating element 61 are $T_{H1}$, $T_{H2}$, and $T_{H3}$, the output voltage $AD_I$ of an A/D converting circuit that is connected to the first temperature measuring element 62 that is in contact with the mixed gas, and the pressure Ps of the mixed gas, as shown in Equation (27), below. if, for example, the A/D converting circuit is of a double integrating type, then the output signal of the A/D converting circuit will be a count value.

$$Q = g[AD_{H1} (T_{H1}), AD_{H2} (T_{H2}), AD_{H3} (T_{H3}), AD_I, Ps ] \dots(27)$$

Moreover, the per-unit-volume calorific value Q of the thermal diffusion rate of a mixed gas can also be obtained from an equation having, as variables, the electric signals $S_{H1}(T_{H1})$, $S_{H2}(T_{H2})$, and $S_{H3}(T_{H3})$ from the heating element 61 when the heat producing temperatures of the heating element 61 are $T_{H1}$, $T_{H2}$, and $T_{H3}$, the electric signal $S_I$ of the first temperature measuring element 62 that is in contact with the mixed gas, and the pressure Ps of the mixed gas, as shown in Equation (28), below.

$$Q = g[S_{H1} (T_{H1}), S_{H2} (T_{H2}), S_{H3} (T_{H3}), S_I, Ps ] \dots(28)$$

The pressure Ps of the mixed gas is measured using a pressure sensor. The pressure sensor includes a strain gauge, for example, made from an electrical resistance element. The strain gauge is deformed by pressure, changing the electric resistance. Consequently, the output pressure from the pressure sensor, or an output signal of an A/D converting circuit that is connected to the output sensor, or the like, is correlated to the pressure Ps of the mixed gas. Consequently, the per-unit-volume calorific value Q of the mixed gas can also be obtained from a formula, as illustrated in Equation (29), below, that uses, as variables, the electric signals $S_{H1}(T_{H1})$, $S_{H2}(T_{H2})$, and $S_{H3}(T_{H3})$ from the heating element 61, the electric signal $S_I$ from the first temperature measuring element 62, and the electric signal Sp from the pressure sensor.

$$Q = g[S_{H1}(T_{H1}), S_{H2}(T_{H2}), S_{H3}(T_{H3}), S_I, S_P] \dots (29)$$

If the temperature of the mixed gas is always the same, then the electric signal $S_I$ from the first temperature measuring element 62 will be a constant. In this case, the per-unit-volume calorific value Q can also be obtained from a formula, as illustrated in Equation (30), below, that uses, as variables, the electric signals $S_{H1}(T_{H1})$, $S_{H2}(T_{H2})$, and $S_{H3}(T_{H3})$ from the heating element 61, and the electric signal $S_P$ from the pressure sensor.

$$Q = g[S_{H1}(T_{H1}), S_{H2}(T_{H2}), S_{H3}(T_{H3}), S_P] \dots (30)$$

The gas components of the mixed gas are not limited to four different components. For example, if the mixed gas comprises n types of gas components, then first a formula, given by Equation (31), below, is obtained using, as variables, the electric signals from the heating element 61 $S_{H1}(T_{H1})$, $S_{H2}(T_{H2})$, $S_{H3}(T_{H3})$, ..., $S_{Hn-1}(T_{Hn-1})$ at least n-1 different the heat producing temperatures $T_{H1}$, $T_{H2}$, $T_{H3}$,..., $T_{Hn-1}$, the electric signal $S_I$ from the first temperature measuring element 62, and the electric signal Sp from the pressure sensor. Given this, the per-unit-volume calorific value Q of the mixed gas to be measured can be calculated uniquely by measuring the values of the electric signals $S_{H1}(T_{H1})$, $S_{H2}(T_{H2})$, $S_{H3}(T_{H3})$, - - -, $S_{Hn-1}(T_{Hn-1})$ from the heating element 61, which contacts the mixed gas to be measured that comprises n different component gases for which the respective volume fractions are unknown, the value of the electric signal $S_I$ from the first temperature measuring element 62, and the value of the electric signal Sp from the pressure sensor that is in contact with the mixed gas to be measured, and then substituting into Equation (31).

$Q = g[S_{H1}(T_{H1}), S_{H2}(T_{H2}), S_{H3}(T_{H3}), ..., S_{Hn-1}(T_{Hn-1}), S_I, Sp] ...(31)$ Note that if the mixed gas includes an alkane ($C_jH_{2j+2}$) other than methane ($CH_4$) and propane ($C_3H_8$), where j is a natural number, in addition to methane ($CH_4$) and propane ($C_3H_8$), then the alkane ($C_jH_{2j+2}$) other than methane ($CH_4$) and propane ($C_3H_8$) will be seen as a mixture of methane ($CH_4$) and propane ($C_3H_8$), and there will be no effect on the calculation in Equation (31). For example, as indicated in Equations (32) through (35), below, the calculation may be performed using Equation (31) by viewing ethane ($C_2H_6$), butane ($C_4H_{10}$), pentane ($C_5H_{12}$), and hexane ($C_6H_{14}$) as a mixture of methane ($CH_4$) and propane ($C_3H_8$), with each multiplied by the respective specific factors.

$$C_2H_6 = 0.5\ CH_4 + 0.5\ C_3H_8 \dots (32)$$

$$C_4H_{10} = -0.5\ CH_4 + 1.5\ C_3H_8 \dots (33)$$

$$C_5H_{12} = -1.0\ CH_4 + 2.0\ C_3H_8 \dots (34)$$

$$C_6H_{14} = -1.5\ CH_4 + 2.5\ C_3H_8 \dots (35)$$

Consequently, with z as a natural number, if a mixed gas comprising n types of gas components includes, as gas components, z types of alkanes ($C_jH_{2j+2}$) other than methane ($CH_4$) and propane ($C_3H_8$), in addition to methane ($CH_4$) and propane ($C_3H_8$), an equation may be calculated having, as variables, the electric signals $S_H$ from the heating element 61 at, at least, n - z-1 different heat producing temperatures, the electric signal $S_I$ from the first temperature measuring element 62, and the electric signal $S_P$ from the pressure sensor.

Note that if the types of gas components in the mixed gas used in the calculation in Equation (31) are the same as the types of gas components of the mixed gas to be measured, wherein the per-unit-volume calorific value Q is unknown, then, of course, Equation (31) can be used in calculating the per-unit-volume calorific value Q of the mixed gas to be measured. Furthermore, Equation (31) can also be used when the mixed gas to be measured comprises a number of gas components that is less than n, where the gas components of the less than n different types are included in the mixed gas that was used for calculating Equation (31). If, for example, the mixed gas used in calculating Equation (31) included four types of gas components, namely methane ($CH_4$), propane ($C_3H_8$), nitrogen ($N_2$) and carbon dioxide ($CO_2$), then even if the mixed gas to be measured includes only three different components, namely methane ($CH_4$), propane ($C_3H_8$), and carbon dioxide ($CO_2$), without containing the nitrogen ($N_2$), still Equation (31) can be used in calculating the calorific value Q of the mixed gas to be measured.

Furthermore, if the mixed gas used in calculating Equation (31) included methane ($CH_4$) and propane ($C_3H_8$) as gas components, Equation (31) could still be used even when the mixed gas to be measured includes an alkane ($C_jH_{2j+2}$) that was not included in the mixed gas that was used in calculating Equation (31). This is because, as described above, even if the alkane ($C_jH_{2j+2}$) other than methane ($CH_4$) and propane ($C_3H_8$) is viewed as a mixture of methane ($CH_4$) and propane ($C_3H_8$) there is no effect on calculating the per-unit-volume calorific value Q using Equation (31).

Moreover, the gas density D is proportional to the calorific value Q of the gas. The calorific value Q of the gas is obtained using Equation (31), above. Consequently, density D of the mixed gas can also be obtained from a formula, as illustrated in Equation (36), below, that uses, as variables, the electric signals $S_H(T_{H1})$, $S_{H2}(T_{H2})$, $S_{H3}(T_{H3})$, ..., $S_{Hn}$-1 ($T_{Hn-1}$) from the heating element 61, the electric signal $S_I$ from the first temperature measuring element 62, and the electric signal Sp from the pressure sensor.

$$D = h[S_{H1}(T_{H1}), S_{H2}(T_{H2}), S_{H3}(T_{H3}), \ldots, S_{Hn-1}(T_Hn-1), S_I, S_P] \ldots (36)$$

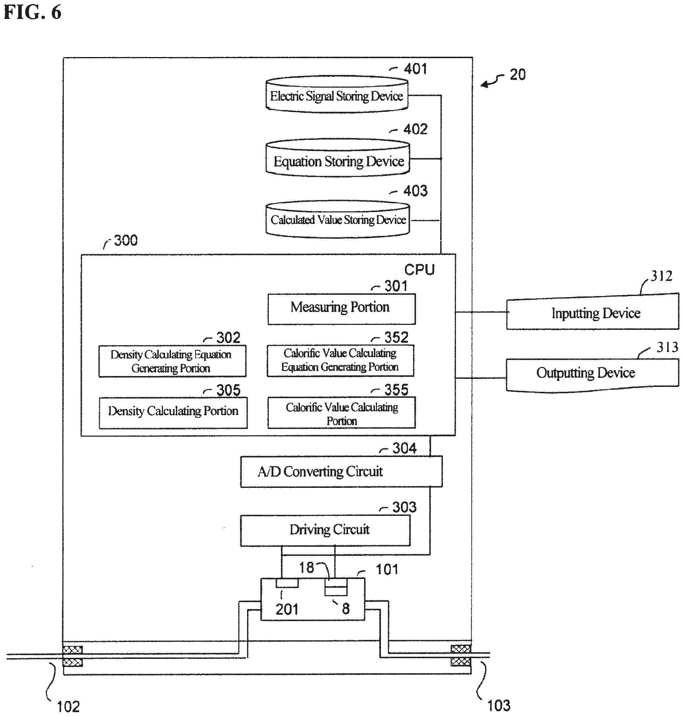

Here the density measuring system 20 according to the form of embodiment illustrated in FIG. 6 comprises: a chamber 101 that is a container into which each of a plurality of sample mixed gases is each injected; and, disposed within the chamber 101: a microchip 8 that includes the first temperature measuring element 62 and the heating element 61 for producing heat at a plurality of heat producing temperatures $T_H$ through the application of a plurality of different voltages thereto, illustrated in FIG. 1; and the pressure sensor 201. Moreover, the density measuring system 20 illustrated in FIG. 6 comprises a measuring portion 301 for measuring the values of the electric signals $S_I$ from the first temperature measuring element 62, which are respectively dependent on the plurality of temperatures $T_I$ of the sample mixed gas, and the value of the electric signal $S_P$ from the pressure sensor 201.

Moreover, the density measuring system 20 further comprises a density calculating equation generating portion 302 and a calorific value calculating equation generating portion 352. Based on values for the density D, values for the electric signal $S_I$ from the first temperature measuring element 62, values for the electric signal $S_H$ from the heating element 61 at a plurality of heat producing temperatures, and values for the electric signal $S_P$ from the pressure sensor 201, known in advance for a plurality of mixed gases, the density calculating equation generating portion 302 generates a density calculating equation that has the electric signal $S_I$ from the first temperature measuring element 62, electric signals $S_H$ from the heating element 61 at a plurality of heat producing temperatures $T_H$, and the electric signal $S_P$ from the pressure sensor 201 as the independent variables, and has the density D of the gas as the dependent variable.

Based on values for the calorific value Q, values for the electric signal $S_I$ from the first temperature measuring element 62, values for the electric signal $S_H$ from the heating element 61 at a plurality of heat producing temperatures, and values for the electric signal $S_P$ from the pressure sensor 201, known in advance for a plurality of mixed gases, the calorific value calculating equation generating portion 352 generates a calorific value calculating equation that has the electric signal $S_I$ from the first temperature measuring element 62, electric signals $S_H$ from the heating element 61 at a plurality of heat producing temperatures $T_H$, and the electric signal $S_P$ from the pressure sensor 201 as the independent variables, and has the calorific value Q as the dependent variable. Note that the sample mixed gasses include a plurality of types of gases.

The microchip 8 is disposed within the chamber 101, by means of a thermally insulating member 18. A flow path 102, for feeding the sample mixed gasses into the chamber 101, and a flow path 103, for discharging the sample mixed gasses from the chamber 101, are connected to the chamber 101.

A gauge pressure sensor or an absolute pressure sensor, for example, can be used as the pressure sensor 201 for measuring the pressure of the gas within the chamber 101. The pressure sensor 201 comprises a pressure-sensitive element. The pressure-sensitive element may use a semiconductor diaphragm type, an electrostatic capacitance type, an elastic diaphragm type, a piezoelectric type, an oscillator type, or the like.

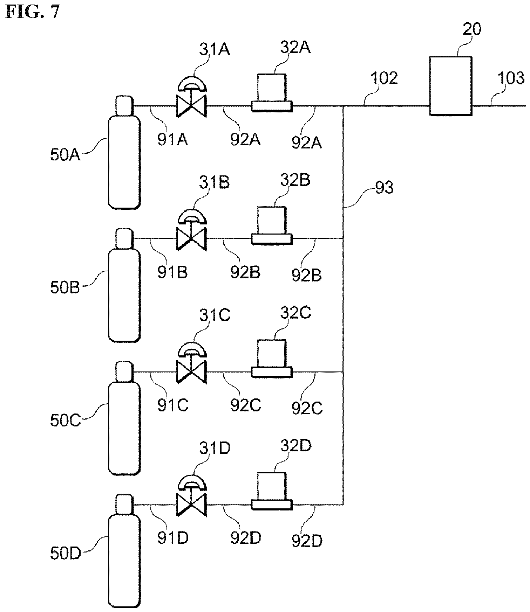

When a four types of sample mixed gases, each having a different density D and calorific value Q, are used, then, as illustrated in FIG. 7, a first gas canister 50A for storing a first sample mixed gas, a second gas canister 50B for storing a second sample mixed gas, a third gas canister 50C for storing a third sample mixed gas, and a fourth gas canister 50D for storing a fourth sample mixed gas are prepared. A first gas pressure regulating device 31A, for controlling the pressure of a first sample mixed gas, is connected through a flow path 91A to a gas canister 50A. Additionally, a first flow rate controlling device 32A is connected through a flow path 92A to the first gas pressure regulating device 31A. The first flow rate controlling device 32A controls the rate of flow of the first sample mixed gas that is fed into density measuring system 20 through the flow paths 92A and 102.

A second gas pressure regulating device 31B is connected through a flow path 91B to the second gas canister 50B. Additionally, a second flow rate controlling device 32B is connected through a flow path 92B to the second gas pressure regulating device 31B. The second flow rate controlling device 32B controls the rate of flow of the second sample mixed gas that is fed into density measuring system 20 through the flow paths 92B, 93, and 102.

A third gas pressure regulating device 31C is connected through a flow path 91C to the third gas canister 50C. Additionally, a third flow rate controlling device 32C is connected through a flow path 92C to the third gas pressure regulating device 31C. The third flow rate controlling device 32C controls the rate of flow of the third sample mixed gas that is fed into density measuring system 20 through the flow paths 92C, 93, and 102.

A fourth gas pressure regulating device $31_D$ is connected through a flow path 91D to the fourth gas canister 50D. Additionally, a fourth flow rate controlling device 32D is connected through a flow path 92D to the fourth gas pressure regulating device 31D. The fourth flow rate controlling device 32D controls the rate of flow of the fourth sample mixed gas that is fed into density measuring system 20 through the flow paths 92D, 93, and 102.

The first through fourth sample mixed gases are each, for example, natural gasses that have different densities and calorific values. The first through fourth sample mixed gases each include four different gas components of, for example, methane ($CH_4$), propane ($C_3H_8$), nitrogen ($N_2$), and carbon dioxide ($CO_2$) at different ratios.

After the first sample mixed gas is filled into the chamber 101 illustrated in FIG. 6, the pressure sensor 201 outputs an electric signal $S_P$ that is dependent on the pressure of the first sample mixed gas. The first temperature measuring element 62 of the microchip 8 illustrated in FIG. 1 and FIG. 2 outputs an electric signal $S_I$ that is dependent on the temperature of the second sample mixed gas. The heating element 61 applies driving powers $P_{H1}$, $P_{H2}$, and $P_{H3}$ from the driving circuit 303 illustrated in FIG. 6. When the driving powers $P_{H1}$ $P_{H2}$, and $P_{H3}$ are applied, the heating element 61 that is in contact with the first sample mixed gas produces heat at a temperature $T_{H1}$ of 100°C, a temperature $T_{H2}$ of 150°C, and a temperature $T_{H3}$ of 200°C, for example, to output an electric signal $S_{H1}$ ($T_{H1}$) at the heat producing temperature $T_{H1}$ an electric signal $S_{H2}$ ($T_{H2}$) at the heat producing temperature $T_{H2}$, and an electric signal $S_{H3}$ ($T_{H3}$) at the heat producing temperature $T_{H3}$.

After the removal of the first sample mixed gas from the chamber 101, the second through fourth sample mixed gases are filled sequentially into the chamber 101. After the second sample mixed gas is filled into the chamber 101, the pressure sensor 201 outputs an electric signal $S_P$ that is dependent on the pressure of the second sample mixed gas. The first temperature measuring element 62 of the microchip 8 illustrated in FIG. 1 and FIG. 2 outputs an electric signal $S_I$ that is dependent on the temperature of the second sample mixed gas. The heating element 61, which is in contact with the second sample mixed gas, outputs an electric signal $S_{H1}$ ($T_{H1}$ at a heat producing temperature $T_{H1}$ an electric signal $S_{H2}$ ($T_{H2}$) at a heat producing temperature $T_{H2}$, and an electric signal $S_{H3}$ ($T_{H3}$) at a heat producing temperature $T_{H3}$.

After the third sample mixed gas is filled into the chamber 101, the pressure sensor 201 outputs an electric signal $S_P$ that is dependent on the pressure of the third sample mixed gas. The first temperature measuring element 62 of the microchip 8 illustrated in FIG. 1 and FIG. 2 outputs an electric signal $S_I$ that is dependent on the temperature of the second sample mixed gas. The heating element 61, which is in contact with the third sample mixed gas, outputs an electric signal $S_{H1}$ ($T_{H1}$) at a heat producing temperature $T_{H1}$ an electric signal $S_{H2}$ ($T_{H2}$) at a heat producing temperature $T_{H2}$, and an electric signal $S_{H3}$ ($T_{H3}$) at a heat producing temperature $T_{H3}$.

After the fourth sample mixed gas is filled into the chamber 101, the pressure sensor 201 outputs an electric signal $S_P$ that is dependent on the pressure of the fourth sample mixed gas. The first temperature measuring element 62 of the microchip 8 illustrated in FIG. 1 and FIG. 2 outputs an electric signal $S_I$ that is dependent on the temperature of the second sample mixed gas. The heating element 61, which is in contact with the fourth sample mixed gas, outputs an electric signal $S_{H1}$ ($T_{H1}$) at a heat producing temperature $T_{H1}$ an electric signal $S_{H2}$ ($T_{H2}$) at a heat producing temperature $T_{H2}$, and an electric signal $S_{H3}$ ($T_{H3}$) at a heat producing temperature $T_{H3}$.

Note that if there are n types of gas components in each of the sample mixed gases, the heating element 61 of the microchip 8, illustrated in FIG. 1 and FIG. 2, is caused to produce heat at at least n-1 different temperatures. However, as described above, an alkane ($C_jH_{2j+2}$) other than methane ($CH_4$) and propane ($C_3H_8$) can be viewed as a mixture of methane ($CH_4$) and propane ($C_3H_8$).Consequently, with z as a natural number, if a sample mixed gas comprising n types of gas components includes, as gas components, z types of alkanes ($C_jH_{2j+2}$) in addition to methane ($CH_4$) and propane ($C_3H_8$), the heating element 61 is caused to produce heat at n-z-1 different temperatures.

As illustrated in FIG. 6, the microchip 8 and the pressure sensor 201 are connected, through an A/D converting circuit 304, to the central calculation processing device (CPU) 300, which includes the measuring portion 301. An electric signal storage device 401 is also connected to the CPU 300. The measuring portion 301 measures the value of the electric signal $S_I$ from the first temperature measuring element 62, and, from the heating element 61, the values of the electric signal $S_{H1}$ ($TH_1$) at the heat producing temperature $T_{H1}$, the electric signal $S_{H2}$ ($T_{H2}$) at the heat producing temperature $T_{H2}$, and the electric signal $S_{H3}$ ($T_{H3}$) at the heat producing temperature $T_{H3}$, and the value of the electric signal $S_P$ from the pressure sensor 201, and stores the measured values in the electric signal storage device 401.

The electric signal $S_I$ from the first temperature measuring element 62 may be the resistance value $R_I$ of the first temperature measuring element 62, the current $I_I$ flowing in the first temperature measuring element 62, the voltage $V_I$ applied to the first temperature measuring element 62, or the output signal $AD_I$ from the A/D converting circuit 304 that is connected to the first temperature measuring element 62. Similarly, the electric signal $S_H$ from the heating element 61 may be the resistance value $R_H$ of the heating element 61, the current $I_H$ flowing in the heating element 61, the voltage $V_H$ applied to the heating element 61, or the output signal $AD_H$ from the A/D converting circuit 304 that is connected to the heating element 61. Moreover, the electric signal $S_P$ from the pressure sensor 201 may be, for example, a resistance value of a strain gauge that is provided in an the pressure sensor 201, an electric current that flows through the strain gauge, a voltage that is applied to the strain gauge, or an output signal from an A/D converting circuit 304 that is connected to the strain gauge.

The density calculating equation generating portion 302 that is included in the CPU 300 collects the respective known values for the densities D of, for example, each of the first through fourth sample mixed gases, the plurality of measured values for the electric signals $S_I$ from the first temperature measuring element 62, the plurality of measured values for the electric signals $S_{H1}$ ($T_{H1}$), $S_{H2}$ ($T_{H2}$), and $S_{H3}$ ($T_{H3}$) from the heating element 61, and the plurality of measured values for the electric signals $S_P$ from the pressure sensor 201. Moreover, the calorific value calculating equation generating portion 352 calculates a calorific value calculating equation, through multivariate statistics, based on the collected values for the densities D, electric signals $S_I$, electric signals $S_H$, and electric signals $S_P$, with the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the pressure sensor 201 as the independent variables and the density D of the gas as the dependent variable.

The calorific value calculating equation generating portion 352 that is included in the CPU 300 collects the respective known values for the calorific values Q of, for example, each of the first through fourth sample mixed gases, the plurality of measured values for the electric signals $S_I$ from the first temperature measuring element 62, the plurality of measured values for the electric signals $S_{H1}$ ($T_{H1}$), $S_{H2}$ ($T_{H2}$), and $S_{H3}$ ($T_{H3}$) from the heating element 61, and the plurality of measured values for the electric signals $S_P$ from the pressure sensor 201. Moreover, the calorific value calculating equation generating portion 352 calculates a calorific value calculating equation, through multivariate statistics, based on the collected values for the calorific values Q, electric signals $S_I$, electric signals $S_H$, and electric signals $S_P$, with the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the pressure sensor 201 as the independent variables and the calorific value Q of the gas as the dependent variable.

Note that "multivariate statistics" includes support vector analysis disclosed in A. J. Smola and B. Scholkopf (eds.), "A Tutorial on Support Vector Regression" (NeuroCOLT Technical Report NC-TR-98-030), multiple linear regression analysis, the Fuzzy Quantification Theory Type II, disclosed in Japanese Unexamined Patent Application Publication H5-141999, and the like.

The density measuring system 20 is further provided with an equation storage device 402, connected to the CPU 300. The equation storage device 402 stores the density calculating equation that is generated by the density calculating equation generating portion 302 and the calorific value calculating equation that is generated by the calorific value calculating equation generating portion 352. An inputting device 312 and an outputting device 313 are also connected to the CPU 300. A keyboard, a pointing device such as a mouse, or the like, may be used as the inputting device 312. An image displaying device such as a liquid crystal display or a monitor, or a printer, or the like, may be used as the outputting device 313.

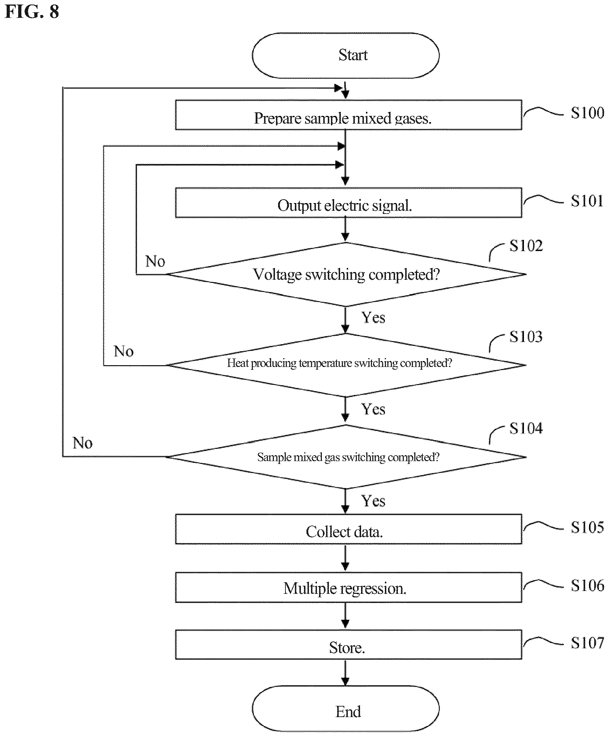

The flowchart shown in FIG. 8 will be used next to explain a method for generating a density calculating equation and a calorific value calculating equation using the density measuring system 20 according to a form of embodiment. Note that in the below an example will be explained wherein the density calculating equation and calorific value calculating equation are generated through setting the pressures within the chamber 101 to atmospheric pressure, 5 kPa, 20 kPa, and 30 kPa.

(a) In Step S 100, the valve for the first flow rate controlling device 32A is opened while leaving the second through fourth flow rate controlling devices 32B through 32D, illustrated in FIG. 7, closed, to introduce the first sample mixed gas into the chamber 101 illustrated in FIG. 6. In Step S101, the pressure within the chamber 101 is put to atmospheric pressure. The measuring portion 301 measures the value of the electric signal $S_P$, indicative of the pressure, from the pressure sensor 201, and stores it in the electric signal storage device 401. Moreover, the measuring portion 301 measures the value of the electric signal $S_I$ from the first temperature measuring element 62 that is in contact with the first sample mixed gas, and stores it in the electric signal storage device 401. Following this, the driving circuit 203 applies a driving power $P_{H1}$ to the heating element 61 illustrated in FIG. 1 and FIG. 2, to cause the heating element 61 produce heat at 100°C. The measuring portion 301, illustrated in FIG. 6, stores, into the electric signal storage device 401, the value of the electric signal $S_{H1}$ ($T_{H1}$) from the heating element 61 that produces heat at 100°C.

(b) In Step S102, the measuring portion 301 evaluates whether or not switching of the gas pressure within the chamber 101 has been completed. If switching to 5 kPa, 20 kPa, and 30 kPa has not been completed, then processing returns to Step S101, and the gas pressure within the chamber 101 is set to 5 kPa. Moreover, at 5 kPa, the measuring portion 301 stores, in the electric signal storage device 401, the value of the electric signal $S_P$ from the pressure sensor 201, the value of the electric signal $S_I$ from the first temperature measuring element 62, and the value of the electric signal $S_{H1}(T_{H1})$ from the heating element 61, which is producing heat at 100°C.

(c) In Step S 102, the measuring portion 301 again evaluates whether or not switching of the gas pressure within the chamber 101 has been completed. If switching to 20 kPa and 30 kPa has not been completed, then processing returns to Step S 101, and the gas pressure within the chamber 101 is set to 20 kPa. Moreover, at 20 kPa, the measuring portion 301 stores, in the electric signal storage device 401, the value of the electric signal $S_P$ from the pressure sensor 201, the value of the electric signal $S_I$ from the first temperature measuring element 62, and the value of the electric signal $S_{H1}(T_{H1})$ from the heating element 61, which is producing heat at 100°C.

(d) In Step S 102, the measuring portion 301 again evaluates whether or not switching of the gas pressure within the chamber 101 has been completed. If switching to 30 kPa has not been completed, then processing returns to Step S101, and the gas pressure within the chamber 101 is set to 30 kPa. Moreover, at 30 kPa, the measuring portion 301 stores, in the electric signal storage device 401, the value of the electric signal $S_P$ from the pressure sensor 201, the value of the electric signal $S_I$ from the first temperature measuring element 62, and the value of the electric signal $S_{H1}(T_{H1})$ from the heating element 61, which is producing heat at 100°C.

(e) If the switching of the pressures within the chamber 101 has been completed, then processing advances from Step S102 to Step S 103. In Step S 103, the driving circuit 303 evaluates whether or not the switching of the temperatures of the heating element 61, illustrated in FIG. 1 and FIG. 2, has been completed. If the switching to the temperature of 150°C and to the temperature of 200°C has not been completed, then processing returns to Step S101, and the driving circuit 303, illustrated in FIG. 6, causes the heating element 61, illustrated in FIG. 1 and FIG. 2, to produce heat at 150°C. Thereafter, Step S 101 and Step S 102 are looped repetitively, and the measuring portion 301 illustrated in FIG. 6 stores, in the electric signal storage device 401, the values for the electric signals $S_P$ from the pressure sensor 201, the values for the electric signals $S_I$ from the first temperature measuring element 62, and the values for the electric signal $S_{H1}(T_{H1})$ from the heating element 61 that is producing heat at 200°C, doing so at atmospheric pressure, 5 kPa, 20 kPa, and 30 kPa.

(f) In Step S 103, the driving circuit 303 again evaluates whether or not the switching of the temperatures of the heating element 61, illustrated in FIG. 1 and FIG. 2, has been completed. If the switching to the temperature of 200°C has not been completed, then processing returns to Step S101, and the driving circuit 303, illustrated in FIG. 6, causes the heating element 61, illustrated in FIG. 1 and FIG. 2, to produce heat at 200°C. Thereafter, Step S 101 and Step S102 are looped repetitively, and the measuring portion 301 illustrated in FIG. 6 stores, in the electric signal storage device 401, the values for the electric signals $S_P$ from the pressure sensor 201, the values for the electric signals $S_I$ from the first temperature measuring element 62, and the values for the electric signal $S_{H1}(T_{H1})$ from the heating element 61 that is producing heat at 200°C, doing so at atmospheric pressure, 5 kPa, 20 kPa, and 30 kPa.

(g) If the switching of the temperature of the heating element 61 has been completed, then processing advances from Step S103 to Step S104. In Step S104, an evaluation is performed as to whether or not the switching of the sample mixed gases has been completed. If the switching to the second through fourth sample mixed gases has not been completed, processing returns to Step S100. In Step S100, the valve for the first flow rate controlling device 32A is closed and the valve for the second flow rate controlling device 32B is opened while leaving the third and fourth flow rate controlling devices 32C through 32D, illustrated in FIG. 7, closed, to introduce the second sample mixed gas into the chamber 101 illustrated in FIG. 6.

(h) The loop of Step S101 through Step S 103 is repeated in the same manner as for the first sample mixed gas. The measuring portion 301 stores, in the electric signal storage device 401, the values for the electric signals $S_P$ from the pressure sensor 201 that is in contact with the second sample mixed gas, the values for the electric signals $S_I$ from the first temperature measuring element 62, and the values for the electric signals $S_{H1}(T_{H1})$, $S_{H2}(T_{H2})$, and $S_{H3}(T_{H3})$ from the heating element 61 that is producing heat at 100°C, 150°C, and 200°C, doing so at atmospheric pressure, 5 kPa, 20 kPa, and 30 kPa.

(i) Thereafter, the loop of Step S100 through Step S104 is repeated. As a result, the measuring portion 301 stores, in the electric signal storage device 401, the values for the electric signals $S_P$ from the pressure sensor 201 that is in contact with the third sample mixed gas, the values for the electric signals $S_I$ from the first temperature measuring element 62, and the values for the electric signals $S_{H1}(T_{H1})$, $S_{H2}(T_{H2})$, and $S_{H3}(T_{H3})$ from the heating element 61 that is producing heat at 100°C, 150°C, and 200°C, doing so at atmospheric pressure, 5 kPa, 20 kPa, and 30 kPa. Moreover, the measuring portion 301 stores, in the electric signal storage device 401, the values for the electric signals $S_P$ from the pressure sensor 201 that is in contact with the fourth sample mixed gas, the values for the electric signals $S_I$ from the first temperature measuring element 62, and the values for the electric signals $S_{H1}(T_{H1})$, $S_{H2}(T_{H2})$, and $S_{H3}(T_{H3})$ from the heating element 61 that is producing heat at 100°C, 150°C, and 200°C, doing so at atmospheric pressure, 5 kPa, 20 kPa, and 30 kPa.

(j) In Step S105, the value for the known density D of the first sample mixed gas, the value for the known density D of the second sample mixed gas, the value for the known density D of the third sample mixed gas, and the value for the known density D of the fourth sample mixed gas are inputted into the density calculating equation generating portion 302 from the inputting device 312. Additionally, the value for the known calorific value Q of the first sample mixed gas, the value for the known calorific value Q of the second sample mixed gas, the value for the known calorific value Q of the third sample mixed gas, and the value for the known calorific value Q of the fourth sample mixed gas are inputted into the calorific value calculating equation generating portion 352 from the inputting device 312. Moreover, the density calculating equation generating portion 302 and the calorific value calculating equation generating portion 352 each read out, from the electric signal storage device 401, the plurality of measured values for the electric signal $S_I$ from the first temperature measuring element 62, the plurality of measured values for the electric signals $S_{H1}(T_{H1})$, $S_{H2}(T_{H2})$, and $S_{H3}(T_{H3})$ from the heating element 61, and the plurality of measured values for the electric signal $S_P$ from the pressure sensor 201.

(k) In Step S106, the density calculating equation generating portion 302 performs a multiple linear regression analysis based on the values for the densities D of, for example, each of the first through fourth sample mixed gases, the plurality of measured values for the electric signals $S_I$ from the first temperature measuring element 62, the plurality of measured values for the electric signals $S_{H1}(T_{H1})$, $S_{H2}(T_{H2})$, and $S_{H3}(T_{H3})$ from the heating element 61, and the plurality of measured values for the electric signals $S_P$ from the pressure sensor 201. Through the multiple linear regression analysis, the density calculating equation generating portion 302 calculates a density calculating equation having the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the temperature sensor 201 as the independent variables and the density D of the gas as the dependent variable. Moreover, the calorific value calculating equation generating portion 352 performs a multiple linear regression analysis based on the values for the calorific values Q of, for example, each of the first through fourth sample mixed gases, the plurality of measured values for the electric signals $S_I$ from the first temperature measuring element 62, the plurality of measured values for the electric signals $S_{H1}(T_{H1})$, $S_{H2}(T_{H2})$, and $S_{H3}(T_{H3})$ from the heating element 61, and the plurality of measured values for the electric signals $S_P$ from the pressure sensor 201. Through the multiple linear regression analysis, the calorific value calculating equation generating portion 352 calculates a calorific value calculating equation having the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the temperature sensor 201 as the independent variables and the calorific value Q of the gas as the dependent variable. Thereafter, in Step S 107, the density calculating equation generating portion 302 stores the generated density calculating equation into the equation storage device 402, the calorific value calculating equation generating portion 352 stores the generated calorific value calculating equation into the equation storage device 402, and the calorific value calculating equation generating method according to the form of embodiment is completed.

As described above, the density calculating equation and calorific value calculating equation generating method that uses the density measuring system 20 according to the example makes it possible to generate a density calculating equation that is able to calculate a unique value for the density D of a gas and a calorific value calculating equation that is able to calculate a unique value for the calorific value Q of the gas.

The functions of a density measuring system 20 when measuring the density D and the calorific value Q of a mixed gas wherein the density D and the calorific value Q are unknown are explained next. For example, a mixed gas to be measured, such as a natural gas that includes, at unknown volume fractions, methane ($CH_4$), propane ($C_3H_8$), nitrogen ($N_2$), and carbon dioxide gas ($CO_2$), with unknown density D and calorific value Q, is introduced into the chamber 101. The pressure sensor 201 outputs an electric signal $S_P$ that depends on the pressure of the mixed gas to be measured. The first temperature measuring element 62 of the microchip 8 illustrated in FIG. 1 and FIG. 2 outputs an electric signal $S_I$ that is dependent on the temperature of the gas that is measured. Following this, the heating element 61 applies driving powers $P_{H1}$, $P_{H2}$, and $P_{H3}$ from the driving circuit 303 illustrated in FIG. 6. When the driving powers $P_{H1}$, $P_{H2}$, and $P_{H3}$ are applied, the heating element 61 that is in contact with the mixed gas being measured produces heat at a temperature $T_{H1}$ of 100°C, a temperature $T_{H2}$ of 150°C, and a temperature $T_{H3}$ of 200°C, for example, to output an electric signal $S_{H1}(T_{H1})$ at the heat producing temperature $T_{H1}$, an electric signal $S_{H2}(T_{H2})$ at the heat producing temperature $T_{H2}$, and an electric signal $S_{H3}(T_{H3})$ at the heat producing temperature $T_{H3}$.

The measuring portion 301, illustrated in FIG. 6, measures the values of the electric signal $S_P$, which is dependent on the pressure of the mixed gas to be measured, from the pressure sensor 201, which is in contact with the mixed gas to be measured, of the electric signal $S_I$, which is dependent on the temperature $T_I$ of the mixed gas to be measured, from the first temperature measuring element 62, and of the electric signal $S_{H1}(T_{H1})$ at the heat producing temperature $T_{H1}$, the electric signal $S_{H2}(T_{H2})$ at the heat producing temperature $T_{H2}$, and the electric signal $S_{H3}(T_{H3})$ at the heat producing temperature $T_{H3}$, from the heating element 61 that is in contact with the mixed gas to be measured, and stores the measured values into the electric signal storage device 401.

As described above, the equation storage device 402 stores a density calculating equation that has, as independent variables, the electric signal $S_I$ from the first temperature measuring element 62, the electric signal $S_{H1}(T_{H1})$ from the heating element 61 with a heat producing temperature $T_{H1}$ of 100°C, the electric signal $S_{H2}(T_{H2})$ from the heating element 61 with a heat producing temperature $T_{H2}$ of 150°C, the electric signal $S_{H3}(T_{H3})$ from the heating element 61 with a heat producing temperature $T_{H3}$ of 200°C, and the electric signal $S_P$ from the pressure sensor 201, and that has, as the dependent variable, the density D of the gas. Moreover, the equation storage device 402 stores a calorific value calculating equation that has, as independent variables, the electric signal $S_I$ from the first temperature measuring element 62, the electric signal $S_{H1}(T_{H1})$ from the heating element 61 with a heat producing temperature $T_{H1}$ of 100°C, the electric signal $S_{H2}(T_{H2})$ from the heating element 61 with a heat producing temperature $T_{H2}$ of 150°C, the electric signal $S_{H3}(T_{H3})$ from the heating element 61 with a heat producing temperature $T_{H3}$ of 200°C, and the electric signal $S_P$ from the pressure sensor 201, and that has, as the dependent variable, the calorific value Q of the gas.

The density measuring system 20 according to an example further includes a density calculating portion 305 and a calorific value calculating portion 355. The density calculating portion 305 substitutes the measured value for the electric value $S_I$ from the first temperature measuring element 62, the measured value for the electric signal $S_H$ from the heating element 61, and the measured value for the electric signal Sp from the pressure sensor 201, respectively, into the independent variable for the electric value $S_I$ from the first temperature measuring element 62, the independent variable for the electric signal $S_H$ from the heating element 61, and the independent variable for the electric signal $S_P$ from the pressure sensor 201, to calculate the measured value for the density D of the mixed gas to be measured, which has been injected into the chamber 101.

The calorific value calculating portion 355 substitutes the measured value for the electric value $S_I$ from the first temperature measuring element 62, the measured value for the electric signal $S_H$ from the heating element 61, and the measured value for the electric signal $S_P$ from the pressure sensor 201, respectively, into the independent variable for the electric value $S_I$ from the first temperature measuring element 62, the independent variable for the electric signal $S_H$ from the heating element 61, and the independent variable for the electric signal $S_P$ from the pressure sensor 201, to calculate the measured value for the calorific value Q of the mixed gas to be measured, which has been injected into the chamber 101.

A calculated value storage device 403 is also connected to the CPU 300. The calculated value storage device 403 stores the value of the density D of the mixed gas to be measured, calculated by the density calculating portion, and the value of the calorific value Q of the mixed gas to be measured, calculated by the calorific value calculating portion 355.

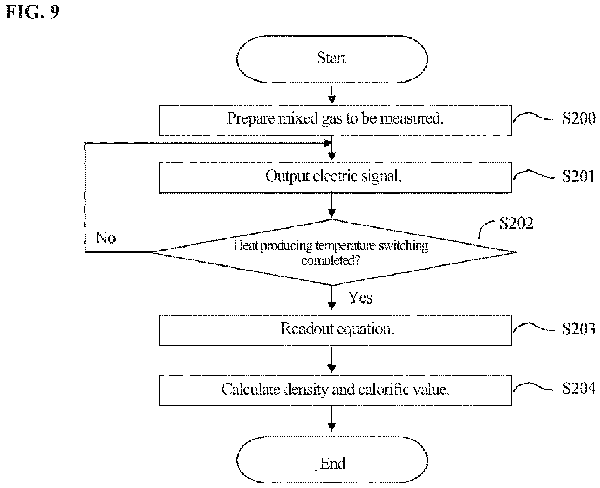

The flowchart shown in FIG. 9 is used next to explain a method for calculating the density and the calorific value using the density measuring system 20 according to another example.

(a) In Step S200, the mixed gas to be measured is introduced into the chamber 101 illustrated in FIG. 6. In Step S201, the measuring portion 301 measures the value of the electric signal Sp from the pressure sensor 201 that is in contact with the mixed gas to be measured, and stores it in the electric signal storage device 401. Moreover, the measuring portion 301 measures the value of the electric signal $S_I$ from the first temperature measuring element 62 that is in contact with the first sample mixed gas, and stores it in the electric signal storage device 401. Following this, the driving circuit 203 applies a driving power $P_{H1}$ to the heating element 61 illustrated in FIG. 1 and FIG. 2, to cause the heating element 61 produce heat at 100°C. The measuring portion 301, illustrated in FIG. 6, stores, into the electric signal storage device 401, the value of the electric signal $S_{H1}$ $(T_{H1})$ from the heating element 61 that is in contact with the mixed gas to be measured and that produces heat at 100°C.

(b) In Step S202, the driving circuit 303, illustrated in FIG. 6, evaluates whether or not the switching of the temperatures of the heating element 61, illustrated in FIG. 1 and FIG. 2, has been completed. If the switching to the temperature of 150°C and to the temperature of 200°C has not been completed, then processing returns to Step S201, and the driving circuit 303 applies a driving power $P_{H2}$ to the heating element 61, illustrated in FIG. 1 and FIG. 2, to cause the heating element 61 to produce heat at 150°C. The measuring portion 301, illustrated in FIG. 6, stores, into the electric signal storage device 401, the value of the electric signal $S_{H2}$ $(T_{H2})$ from the heating element 61 that is in contact with the mixed gas to be measured and that produces heat at 150°C.

(c) In Step S202, whether or not the switching of the temperatures of the heating element 61, illustrated in FIG. 1 and FIG. 2, has been completed is evaluated again. If the switching to the temperature of 200°C has not been completed, then processing returns to Step S201, and the driving circuit 303 applies a driving power $P_{H3}$ to the heating element 61, illustrated in FIG. 1 and FIG. 2, to cause the heating element 61 to produce heat at 200°C. The measuring portion 301, illustrated in FIG. 6, stores, into the electric signal storage device 401, the value of the electric signal $S_{H3}$ $(T_{H3})$ from the heating element 61 that is in contact with the mixed gas to be measured and that produces heat at 200°C.

(d) If the switching of the temperature of the heating element 61 has been completed, then processing advances from Step S202 to Step S203. In Step S203, the density calculating portion 305, illustrated in FIG. 6, reads out, from the equation storage device 402, a density calculating equation having the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the temperature sensor 201 as the independent variables and the density D of the gas as the dependent variable. Moreover, the calorific value calculating portion 355 reads out, from the equation storage device 402, a calorific value calculating equation having the electric signal $S_I$ from the first temperature measuring element 62, the electric signals $S_{H1}(T_{H1})$, $S_{H2}(T_{H2})$, and $S_{H3}(T_{H3})$ from the heating element 61, and the electric signal $S_P$ from the temperature sensor 201 as the independent variables and the calorific value Q of the gas as the dependent variable. Moreover, the density calculating portion 305 and the calorific value calculating portion 355 each read out, from the electric signal storage device 401, a measured value for the electric signal $S_I$ from the first temperature measuring element 62 that is in contact with the mixed gas to be measured, measured values for the electric signals $S_{H1}(T_{H1})$, $S_{H2}(T_{H2})$, and $S_{H3}(T_{H3})$ from the heating element 61 that is in contact with the mixed gas to be measured, and a measured value for the electric signal $S_P$ from the pressure sensor 201.

(e) In Step S204, the density calculating portion 305 substitutes the respective measured values into the independent variables for the electric signal $S_I$, the electric signals $S_{H1}(T_{H1})$, $S_{H2}(T_{H2})$, and $S_{H3}(T_{H3})$, and the electric signal $S_P$ of the density calculating equation, to calculate the value of the density D of the mixed gas to be measured. Additionally, the calorific value calculating portion 355 substitutes the respective measured values into the independent variables for the electric signal $S_I$, the electric signals $S_{H1}(T_{H1})$, $S_{H2}(T_{H2})$, and $S_{H3}(T_{H3})$, and the electric signal $S_P$ of the calorific value calculating equation, to calculate the value of the calorific value Q of the mixed gas to be measured. Thereafter, the density calculating portion 305 stores the calculated value for the density D into the calculated value storage device 403, and the calorific value calculating portion 355 stores the calculated value for the calorific value Q into the calculated value storage device 403, to complete the density measuring method according to the example.

The calorific value calculating method according to the example explained above make it possible to measure the value of the density D and the value of the calorific value Q of a mixed gas to be measured, from the value of the electric signal $S_I$ from the first temperature measuring element 62 that is in contact with the mixed gas to be measured, the values of the electric signals $S_{H1}(T_{H1})$, $S_{H2}(T_{H2})$, $S_{H3}(T_{H3})$ from the heating element 61 that is in contact with the mixed gas to be measured, and the value of the electric signal $S_P$ from the pressure sensor 201 that is in contact with the mixed gas to be measured.

The hydrocarbon compositional ratios of natural gas vary depending on the gas fields from which it is produced. Moreover, natural gas also includes nitrogen ($N_2$) and carbon dioxide gas ($CO_2$), and the like, in addition to the hydrocarbons. Because of this, the volume fractions of the gas components that are included in the natural gas will vary depending on the gas field of production, and even if the types of the gas components are known in advance, often the density D and the calorific value Q of the natural gas are unknown. Moreover, even with natural gas that derives from the same gas field, the densities D and calorific values Q are not always identical, and may vary depending on the timing of extraction.

Conventionally, when collecting natural gas usage fees, a method was used wherein the charges would be calculated based on the volume used, rather than on the calorific value Q of the natural gas used. However, because the calorific value Q varies depending on the gas field of production, from which the natural gas is derived, it is not fair to charge based on the volume used. In contrast, the use of the calorific value calculating method according to the present example it possible to calculate easily the density D and the calorific value Q of a mixed gas, such as a natural gas, wherein the types of the gas components are known in advance but the density D and the calorific value Q are not known because the volume fractions of the gas components are not known. This makes it possible to charge fair usage fees.

Moreover, release into the atmosphere of gas for which the density D and calorific value in Q has been measured is undesirable from an environmental perspective. Consequently, when measuring the density D and the calorific value Q of gas in a gas pipe, preferably the density measuring system is provided in the gas pipe itself or the density measuring system is provided in a bypass route from the gas pipe, and preferably the gas for which the density D and the calorific value Q have been measured is returned to the gas pipe. At this time, the gas pressure within the gas pipe may vary substantially. In this regard, the density measuring system according to the present example makes it possible to suppress the calculation error in the density D and the calorific value in Q due to the variability of the pressure, through including the pressure as an independent variable in the calorific value calculating equation. Note that there is no need for the pressure sensor 201 to have a correcting circuit if both the generation of the calorific value calculating equation and the measurement of the calorific value use the same pressure sensor 201. This is because it is possible to suppress calculation error in the calorific value due to variability in the pressure through merely measuring the electric signals that are outputted from the pressure sensor 201 in response to the pressure, even if the value of the pressure is not necessarily measured accurately.

While there are descriptions of examples set forth above, the descriptions and drawings that form a portion of the disclosure are not to be understood to limit the present invention. A variety of alternate forms of embodiment and operating technologies should be obvious to those skilled in the art. For example, in the present example the explanation was for a case wherein the equation storage device 402, illustrated in FIG. 6, stored a density calculating equation wherein an electric signal from the pressure sensor 201, an electric signal from a first temperature measuring element 62, illustrated in FIG. 1, and electric signals from a heating element 61 at a plurality of different heat producing temperatures are the independent variables and the density D is the dependent variable.

In contrast, as explained in Equation (23), above, the calorific value Q, which is proportional to the density D of the gas, can be obtained from an equation wherein the pressure $P_S$ of the gas and the radiation coefficients $M_{I1}(T_{H1})$, $M_{I2}(T_{H2})$, and $M_{I3}(T_{H3})$ of the gas at the respective temperatures $T_{H1}$, $T_{H2}$, and $T_{H3}$ for the heating element 61 are the variables. As a result, the equation storage device 402, illustrated in FIG. 6, may store a density calculating equation wherein the pressure of the gas and the radiation coefficients of the gas at a plurality of heat producing temperatures of the heating element 61 are the independent variables and the density D is the dependent variable. In this case, the measuring portion 301 measures the measured values for the radiation coefficients of the gas that is injected into the chamber 101, doing so with the heating element 61 producing heat at a plurality of heat producing temperatures. Note that as was explained for Equation (9), above, it is possible to measure the radiation coefficients of the gas using a microchip 8. The density calculating portion 305 substitutes the measured value for the pressure of the gas and the measured values for the radiation coefficients of the gas into the independent variables in the density calculating equation stored in the equation storage device 402, to calculate the measured value for the density D of the gas.

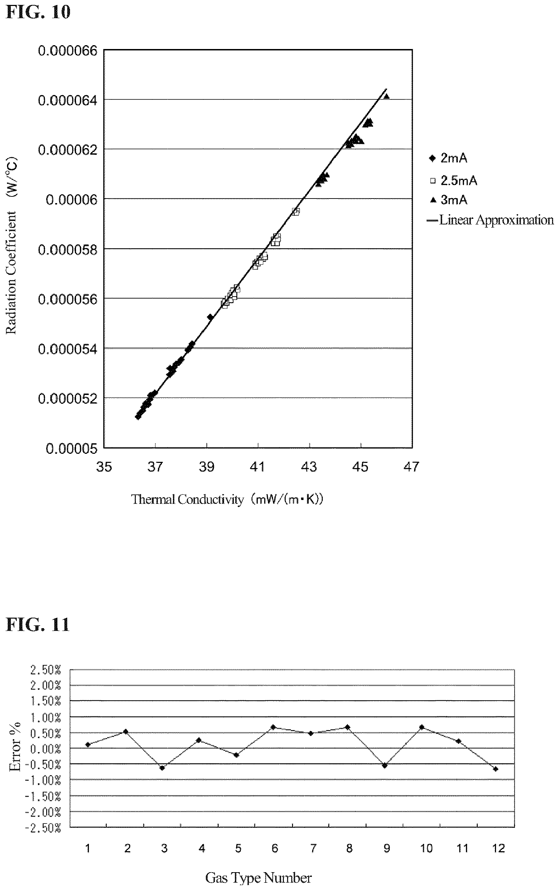

The relationship between the radiation coefficient and the thermal conductivity in a mixed gas when electric currents of 2 mA, 2.5 mA, and 3 mA are produced in a heat producing resistance is explained next. As illustrated in FIG. 10, typically there is a proportional relationship between the radiation coefficient and the thermal conductivity of the mixed gas. As a result, the equation storage device 402, illustrated in FIG. 6, may store a density calculating equation wherein the pressure of the gas and the thermal conductivities of the gas at a plurality of heat producing temperatures of the heating element 61 are the independent variables and the density D is the dependent variable. In this case, the measuring portion 301 measures the measured values for the thermal conductivities of the gas that is injected into the chamber 101, doing so with the heating element 61 producing heat at a plurality of heat producing temperatures. The calorific value calculating portion 355 substitutes the measured value for the pressure of the gas and the measured values for the thermal conductivities of the gas into the independent variables in the density calculating equation stored in the equation storage device 402, to calculate the measured value for the density D of the gas.

In this way, the present invention should be understood to include a variety of examples, and the like, not set forth herein.

First, 12 different sample mixed gases with known values for the calorific value Q were prepared. The 12 different sample mixed gases each included methane ($CH_4$), propane ($C_3H_8$), nitrogen ($N_2$), and/or carbon dioxide gas ($CO_2$) as gas components. For example, a particular sample mixed gas included 90 vol% methane, 3 vol% ethane, 1 vol% propane, 1 vol% butane, 4 vol% nitrogen, and 1 vol% carbon dioxide. Moreover, a particular sample mixed gas included 85 vol% methane, 10 vol% ethane, 3 vol% propane, and 2 vol% butane, and did not include nitrogen or carbon dioxide. Moreover, a particular sample mixed gas included 85 vol% methane, 8 vol% ethane, 2 vol% propane, 1 vol% butane, 2 vol% nitrogen, and 2 vol% carbon dioxide.

Following this, each of the 12 different sample mixed gases were used to obtain a plurality of measured values for the electric signal $S_P$ from the pressure sensor, and a plurality of measured values for the electric signals $S_{H1}(T_{H1})$, $S_{H2}(T_{H2})$, $S_{H3}(T_{H3})$, and $S_{H4}(T_{H4})$ from the heating element to which four different voltages were applied. Thereafter, an equation for calculating the density D was produced through support vector regression, based on the known values for the densities D of the 12 different sample mixed gases, the plurality of measured values for the electric signals $S_P$ from the pressure sensor, and the plurality of measured values for the electric signals $S_{H1}(T_{H1})$, $S_{H2}(T_{H2})$, $S_{H3}(T_{H3})$, and $S_{H4}(T_{H4})$ from the heating element 61, with the electric signal $S_P$ from the pressure sensor and the values for the electric signals $S_{H1}(T_{H1})$, $S_{H2}(T_{H2})$, $S_{H3}(T_{H3})$, and $S_{H4}(T_{H4})$ from the heating element as the independent variables and the density D as the dependent variable.

The generated equation for calculating the density D was used to calculate the respective densities D of the 12 different sample mixed gases, and when compared to the true densities D, the error was within a range of $\pm$ 0.65%, as illustrated in FIG. 11.

Following this, each of the 12 different sample mixed gases were used to obtain a plurality of measured values for the electric signal $S_P$ from the pressure sensor, and a plurality of measured values for the electric signals $S_{H1}(T_{H1})$, $S_{H2}(T_{H2})$, $S_{H3}(T_{H3})$, and $S_{H4}(T_{H4})$ from the heating element to which four different voltages were applied. Thereafter, an equation for calculating the calorific value Q was produced through support vector regression, based on the known values for the calorific values Q of the 12 different sample mixed gases, the plurality of measured values for the electric signals $S_P$ from the pressure sensor, and the plurality of measured values for the electric signals $S_{H1}(T_{H1})$, $S_{H2}(T_{H2})$, $S_{H3}(T_{H3})$, and $S_{H4}(T_{H4})$ from the heating element 61, with the electric signal $S_P$ from the pressure sensor and the values for the electric signals $S_{H1}(T_{H1})$, $S_{H2}(T_{H2})$, $S_{H3}(T_{H3})$, and $S_{H4}(T_{H4})$ from the heating element as the independent variables and the calorific value Q as the dependent variable.

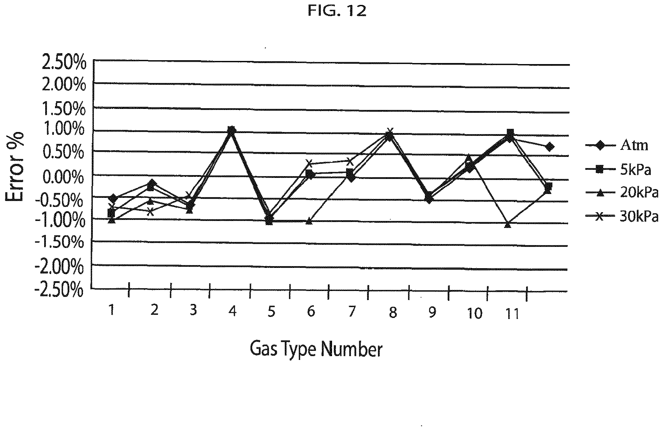

The generated was used to calculate the respective calorific values Q of the 12 different sample mixed gases, and when compared to the true calorific values Q, the error was within a range of $\pm$ 1%, as illustrated in FIG. 12.

**Claims**

1.  A density measuring system (20), comprising:

    a measuring portion (301) measuring a measured value of at least a gas radiation coefficient or a thermal conductivity;
    a storage device storing a correlation between at least a radiation coefficient or a thermal conductivity and a density; and
    a density calculating portion (305) calculating a measured value for a density of the gas based on at least the measured value for the gas radiation coefficient or thermal conductivity and the correlation.

2.  The density measuring system as set forth in Claim 1, wherein:

    the measuring portion (301) measures a measured value for a pressure of the gas;
    the correlation includes a relationship having a pressure of a gas; and
    the density calculating portion (305) calculates a measured value for a density of a gas based on at least one of the measured value for the radiation coefficient or thermal conductivity the gas, the measured value for the pressure, and the correlation.

3.  The density measuring system as set forth in Claim 1 or 2, wherein:

    the correlation is based on density values for a plurality of sample mixed gases that include a plurality of types of gas components, and respective values for the radiation coefficients or thermal conductivities of the plurality of sample mixed gases.

4.  The density measuring system as set forth in Claim 3, wherein:

    the correlation is obtained by support vector regression.

5.  The density measuring system as set forth in anyone of the preceding claims, further comprising:

    a calorific value calculating portion (355) calculating a measured value for the calorific value of a gas injected into the measuring portion, based on a correlation relating to a measured value of at least the radiation coefficient or the thermal conductivity of the gas and the calorific value, where the memory device further stores at least a correlation the radiation coefficient or the thermal conductivity and the calorific value.

6.  The density measuring system as set forth in Claim 5, wherein: the correlation regarding the calorific value is based on calorific values for a plurality of sample mixed gases that include a plurality of types of gas components, and respective values for the radiation coefficients or thermal conductivities of the plurality of sample mixed gases.

7.  The density measuring system as set forth in Claim 6, wherein:

    the correlation is obtained by support vector regression.

8.  A method for measuring a density of a gas, comprising the steps of:

    measuring at least one of a gas radiation coefficient or a gas thermal conductivity;
    preparing a correlation between at least one of the radiation coefficient or the thermal conductivity and a density; and
    calculating a measured value density of the gas based on at least one of the radiation coefficient or the thermal conductivity the gas and the correlation.

9.  The method for measuring a density as set forth in Claim 8, further comprising the step of:

measuring the pressure of the gas; wherein the calculating step calculates the measured value density of the gas based on at least one of the radiation coefficient, the thermal conductivity the gas, or the pressure, and the correlation.

10. The method for measuring a density as set forth in Claim 8 or 9, further comprising the step of obtaining the correlation based on density values for a plurality of sample mixed gases that include a plurality of types of gas components, and respective values for radiation coefficients or thermal conductivities of the plurality of sample mixed gases.

11. The method for measuring a density as set forth in Claim 10, wherein:

the obtaining step includes using support vector regression to obtain the correlation.

12. The method for measuring a density as set forth in anyone of the Claims 8 to 11, further comprising the steps of:

preparing a correlation between at least a radiation coefficient or a thermal conductivity and a calorific value; and
calculating a calorific value of the gas based on at least the radiation coefficient or the thermal conductivity the gas and the correlation regarding the calorific value.

13. The method for measuring a density as set forth in Claim 12, further comprising the step of obtaining the correlation regarding the calorific value based on calorific values for a plurality of sample mixed gases that include a plurality of types of gas components, and respective values for radiation coefficients or thermal conductivities of the plurality of sample mixed gases.

14. The method for measuring a density as set forth in Claim 13, wherein:

the obtaining step includes using support vector regression to obtain the correlation relating to the calorific value.

**FIG. 1**

**FIG. 2**

**FIG. 3**

**FIG. 4**

FIG. 5

**FIG. 6**

FIG. 7

**FIG. 8**

```
                    ┌─────────────┐
                    │    Start    │
                    └─────────────┘
                           │
    ┌──────────────────────┤
    │  ┌────────────────────────────────────────┐
    │  │      Prepare sample mixed gases.        │────── S100
    │  └────────────────────────────────────────┘
    │  ┌───────────────────────┤
    │  │  ┌──────────────────────┤
    │  │  │  ┌────────────────────────────────────────┐
    │  │  │  │         Output electric signal.         │────── S101
    │  │  │  └────────────────────────────────────────┘
    │  │  │                     │
    │  │  │  No       ◇─────────────────────◇          S102
    │  │  └─────────◇  Voltage switching completed? ◇
    │  │             ◇─────────────────────◇
    │  │                       │ Yes
    │  │  No       ◇─────────────────────────◇          S103
    │  └─────────◇ Heat producing temperature ◇
    │             ◇  switching completed?      ◇
    │             ◇─────────────────────────◇
    │                       │ Yes
    │  No       ◇──────────────────────────◇          S104
    └─────────◇ Sample mixed gas switching   ◇
              ◇       completed?              ◇
              ◇──────────────────────────◇
                          │ Yes
              ┌────────────────────────────────────────┐
              │              Collect data.              │────── S105
              └────────────────────────────────────────┘
                          │
              ┌────────────────────────────────────────┐
              │           Multiple regression.          │────── S106
              └────────────────────────────────────────┘
                          │
              ┌────────────────────────────────────────┐
              │                 Store.                  │────── S107
              └────────────────────────────────────────┘
                          │
                    ┌─────────────┐
                    │     End     │
                    └─────────────┘
```

**FIG. 9**

```
              ┌─────────────┐
              │    Start    │
              └──────┬──────┘
                     ↓
   ┌─────────────────────────────────────┐
   │   Prepare mixed gas to be measured.  │──── S200
   └─────────────────┬───────────────────┘
    ┌──────────────→ ↓
   ┌─────────────────────────────────────┐
   │        Output electric signal.        │──── S201
   └─────────────────┬───────────────────┘
    │                ↓                                  S202
   No    ◇───────────────────────────────◇
    │    ╱  Heat producing temperature switching  ╲
    └────        completed?
         ╲                                 ╱
          ◇───────────┬─────────────────◇
                      ↓  Yes
   ┌─────────────────────────────────────┐
   │           Readout equation.           │──── S203
   └─────────────────┬───────────────────┘
                     ↓
   ┌─────────────────────────────────────┐
   │  Calculate density and calorific value. │──── S204
   └─────────────────┬───────────────────┘
                     ↓
              ┌─────────────┐
              │     End     │
              └─────────────┘
```

## FIG. 10

## FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011066579 A
- JP H10281967 B

- JP H5141999 B

**Non-patent literature cited in the description**

- A Tutorial on Support Vector Regression. Neuro-COLT Technical Report NC-TR-98-030